(19) Europäisches Patentamt

European Patent Office

Office européen des brevets

(11) Veröffentlichungsnummer: **0 406 390 B1**

(12) **EUROPÄISCHE PATENTSCHRIFT**

(45) Veröffentlichungstag der Patentschrift: **13.04.94**

(21) Anmeldenummer: **90901816.0**

(22) Anmeldetag: **22.01.90**

(86) Internationale Anmeldenummer:
**PCT/EP90/00123**

(87) Internationale Veröffentlichungsnummer:
**WO 90/07941 (26.07.90 90/17)**

(51) Int. Cl.⁵: **A61L 25/00**

(54) **OPERATIONSNADEL UND VERFAHREN ZU IHRER HERSTELLUNG.**

(30) Priorität: **20.01.89 DE 3901643**
**11.04.89 DE 3911878**
**14.07.89 DE 3923346**
**15.09.89 DE 3930921**

(43) Veröffentlichungstag der Anmeldung:
**09.01.91 Patentblatt 91/02**

(45) Bekanntmachung des Hinweises auf die
Patenterteilung:
**13.04.94 Patentblatt 94/15**

(84) Benannte Vertragsstaaten:
**AT BE CH DE DK ES FR GB IT LI LU NL SE**

(56) Entgegenhaltungen:
**EP-A- 0 208 172**
**EP-A- 0 340 016**

(73) Patentinhaber: **Schumann, Klaus, Prof. Dr. med.**
**Franz-Wiedemeier-Strasse 27**
**D-89081 Ulm(DE)**

(72) Erfinder: **Schumann, Klaus, Prof. Dr. med.**
**Franz-Wiedemeier-Strasse 27**
**D-89081 Ulm(DE)**

(74) Vertreter: **Zinnecker, Armin, Dipl.-Ing. et al**
**Lorenz-Seidler-Gossel,**
**Widenmayerstrasse 23**
**D-80538 München (DE)**

**Beschreibung**

Die Erfindung betrifft eine Operationsnadel für die Mittelohr- und Unfallchirurgie, wie zur Behandlung von Schädelfrakturen, die Rekonstruktion der hinteren Gehörgangswand sowie insbesondere die Mittelohrchirurgie zur Überbrückung von Gehörknöchelchendefekten und ein Verfahren zu ihrer Herstellung.

Die Verwendung von Glasionomer-Zementen in der Zahnheilkunde ist bekannt. Glasionomer-Zemente bestehen im allgemeinen aus einem pulverförmigen Calciumaluminiumfluorsilikatglas und einem Carbonsäure-Polymer oder -Copolymer. Zur Abbindung wird Weinsäure als Starter zugesetzt. Die herausragenden Eigenschaften der Glasionomer-Zemente sind neben einer guten Verträglichkeit im vitalen Gewebe eine signifikante Haftung am Zahnmaterial, wie Schmelz und Dentin, oder Knochen. Die bei der Verwendung von Glasionomer-Zement in der Zahnheilkunde üblicherweise getätigte Zubereitung erfolgt mit einem Rüttler und einer speziellen Spritze. Diese Art der Zubereitung hat sich jedoch für andere Anwendungen als nicht geeignet herausgestellt.

Der Erfindung liegt daher die Aufgabe Zugrunde, Glasionomer-Zement in einer Zubereitung, welche bei anderen Anwendungen als in der Zahnheilkunde, wie in der Mittelohr- und Unfallchirurgie, bei der Rekonstruktion der hinteren Gehörgangswand und insbesondere in der Mittelohrchirurgie zur Überbrückung von Gehörknöchelchendefekten, vorteilhaft anwendbar ist, und ein Verfahren zu deren Herstellung zu schaffen.

Dies wurde überraschenderweise durch die Erfindung erreicht.

Es wurde festgestellt, daß die in der Zahnheilkunde eingesetzten Zubereitungen den Nachteil haben, daß ihre Fließeigenschaften für andere Anwendungen als in der Zahnheilkunde nicht gut genug sind und ihre Empfindlichkeit gegenüber Feuchtigkeit, Wasser und wäßrigen Flüssigkeiten zu gering ist. Bei den aus der Zahnchirurgie bekannten Zubereitungen, deren Herstellung mit einem Rüttler und einer speziellen Spritze erfolgte, konnte nämlich das Material nur gespachtelt werden, es hatte keine Fließeigenschaft und war extrem empfindlich auf Feuchtigkeit, was zum Beispiel bei Schädelbasisrevisionen infolge Liquorfluß extrem nachteilig ist.

Nun wurde überraschenderweise festgestellt, daß im Falle einer solchen Zubereitung, welche aus einer Operationsnadel mit einem an ihr hängenden Tropfen einer wäßrigen Anmischung eines zu Glasionomer-Zement erhärtbaren Glasionomer-Materiales besteht, die Fließeigenschaften bedeutend verbessert und die Empfindlichkeit gegenüber Feuchtigkeit, Wasser und wäßrigen Flüssigkeiten bedeutend verringert sind. Daher können solche Operationsnadeln mit ausgezeichnetem Erfolg in der Mittelohrchirurgie, in der Unfallchirurgie, insbesondere der Chirurgie im Bereich des Gesichtes und des Schädels, ganz besonders bei der Revision von Schädelbasisfrakturen, sowie bei der Rekonstruktion der hinteren Gehörgangswand und insbesondere bei der Mittelohrchirurgie zur Überbrückung von Gehörknöchelchendefekten eingesetzt werden.

Gegenstand der Erfindung ist daher eine Operationsnadel für die Mittelohr- und Unfallchirurgie sowie die Rekonstruktion der hinteren Gehörgangswand, welche dadurch gekennzeichnet ist, daß an ihr ein Tropfen einer wäßrigen Anmischung eines zu Glasionomer-Zement erhärtbaren Glasionomer-Materiales hängt.

Die Anwendung dieser Operationsnadel kann in der Weise erfolgen, daß der Operateur die Operationsnadel mit dem Tropfen zum zu versorgenden Gebiet bringt und wartet, bis erausgehärtet ist. Der Assistent meldet an Hand des angerührten Restes den Zeitpunkt, zu welchem die Aushärtung beendet ist. Nach dieser Meldung überprüft der Operateur das Operationsergebnis.

Vorzugsweise beträgt an der erfindungsgemäßen Operationsnadel die Tropfengröße 0,5 bis 2,0 mm Durchmesser.

Es ist auch bevorzugt, daß das Glasionomer-Material der erfindungsgemäßen Operationsnadel aus pulverförmigem Calciumaluminiumsilikatglas und/oder Calciumaluminiumfluorsilikatglas, gegebenenfalls mit einem Gehalt an Natrium und/oder Phosphor, aus einem Polymer und/oder Copolymer einer Carbonsäure und aus einem Chelatbildner als Abbindestarter besteht. Das Vorstehende ist so zu verstehen, daß von den einzelnen Bestandteilen auch jeweils mehr als 1 vorliegen kann.

Sehr vorteilhaft sind Natriumcalciumaluminiumfluorsilikatgläser. Besonders bevorzugtbesteht das Calciumaluminiumfluorsilikatglas aus

150 bis 200 Gew.-Teilen $SiO_2$ ,
75 bis 125 Gew.-Teilen $Al_2O_3$ ,
180 bis 240 Gew.-Teilen $CaF_2$ ,
20 bis 40 Gew.-Teilen $Na_3AlF_6$ ,
25 bis 40 Gew.-Teilen $AlF_3$ und
50 bis 70 Gew.-Teilen $AlPO_4$ .

Eine beispielhafte Zusammensetzung ist wie folgt.
175 Gew.-Teile $SiO_2$ ,
100 Gew.-Teile $Al_2O_3$ ,
207 Gew.-Teile $CaF_2$ ,
30 Gew.-Teile $Na_3AlF_6$ ,
32 Gew.-Teile $AlF_3$ ,
60 Gew.-Teile $AlPO_4$

Vorzugsweise ist das Polymer einer ungesättigten Carbonsäure, gegebenenfalls halogensubstituierte, Polyacrylsäure, gegebenenfalls halogensubstituierte, Polymethacrylsäure oder Polymaleinsäure und das Copolymer einer ungesättigten Carbonsäure ein solches von Acrylsäure und Methacryksäure, Acrylsäure und Itaconsäure oder Acrylsäure und Maleinsäure oder Methacrylsäure und Itaconsäure oder Methacrylsäure und Maleinsäure oder Acrylsäure, Methacrylsäure und Itaconsäure oder Acrylsäure, Methacrylsäure und Maleinsäure oder Acrylsäure, Itaconsäure und Malinsäure oder Methacrylsäure, Itaconsäure und Maleinsäure oder Acrylsäure, Methacrylsäure, Itaconsäure und Maleinsäure, die alle gegebenenfalls halogensubstituiert sein können. Von den Polymeren einer ungesättigten Carbonsäure sind die von Acrylsäure und Methacrylsäure bevorzugt, ganz besonders die ersteren.

Beispiele für weitere Comonomere sind Acrylamid, Acrylnitril, 2-(Hydroxy)-methacrylat, 2-(Chlor)-acrylsäure, 3-(Chlor)-acrylsäure, 2-(Brom)-acrylsäure, 3-(Brom)-acrylsäure, Glutaconsäure, Aconitsäure, Tiglinsäure, Fumarsäure, Mesaconsäure und Citraconsäure.

Vorzugsweise ist der als Abbindestarter dienende Chelatbildner ein solcher mit 1 oder mehr Carboxyl- und/oder Hydroxygruppe(n), insbesondere in einer Alkylkette. Besonders bevorzugt ist er Weinsäure. Weitere Beispiele sind Mesoweinsäure, Citronensäure, Milchsäure, Äpfelsäure, Glykolsäure, Äthylendiamintetraessigsäure, Salicylsäure, 2,4- und 2,6-Dihydroxybenzoesäuren, Dihydroxyweinsäure, Nitrilotriessigsäure, Mellithsäure und Polyglykole. Von den Carbonsäuren sind auch deren Salze, insbesondere Alkalisalze, umfaßt.

Alternativ bevorzugt ist der als Abbindestarter dienende Chelatbildner ein, insbesondere 2- oder 3-wertiges, Metallchelat. Beispiele für solche sind Komplexe von $\beta$-Diketonen mit Aluminium und Chrom, wie Aluminium- und Chromtriacetylacetonate, und Äthylendiamintetraessigsäurekomplexe von Zink und Kupfer.

Nach einer vorteilhaften Ausführungsform der erfindungsgemäßen Operationsnadel besteht die wäßrige Anmischflüssigkeit des Glasionomer-Materiales aus

| | |
|---|---|
| 35 bis 45 Gew.-%, insbesondere 40 bis 42 Gew.-%,ganz besonders 41,5 Gew.-%, | Calciumaluminiumsilikatglas und/oder Calciumaluminiumfluorsilikatglas, gegebenenfalls mit einem Gehalt an Natrium- und/oder Phosphor, |
| 5 bis 15 Gew.-%, insbesondere 8 bis 10 Gew.-%, ganz besonders 8,5 Gew.-%, | Polymer und/oder Copolymer einer ungesättigten Carbonsäure, |
| 5 bis 15 Gew.-%, insbesondere 8 bis 10 Gew.-%, ganz besonders 8,5 Gew.-%, | als Abbindestarter dienenden Chelatbildner |
| | und |
| 35 bis 45 Gew.-%, insbsondere 40 bis 42 Gew.-, ganz besonders 41,5 Gew.-%, | Wasser |

Nach einer besonders bevorzugten speziellen Ausführungsform der erfindungsgemäßen Operationsnadel ist das Calciumaluminiumfluorsilikatglas ein Natriumcalciumaluminiumfluorsilikat-Glas, das Copolymer einer ungesättigten Carbonsäure Poly-(acryl/malein)-säure, vorzugsweise mit einem Verhältnis der Acrylsäure- und Maleinsäureeinheiten von 1 : 1, und der als Abbindestarter dienende Chelatbildner Weinsäure.

Gegenstand der Erfindung ist auch ein Verfahren zur Herstellung der erfindungsgemäßen Operationsnadel, welches dadurch gekennzeichnet ist, daß das pulverförmige Calciumaluminiumsilikatglas und/oder Calciumaluminiumfluorsilikatglas, das Polymer und/oder Copolymer einer ungesättigten Carbonsäure und der als Abbindestarter dienende Chelatbildner in Wasser mit einer Operationsnadel angerührt werden, bis an der letzteren ein Tropfen hängen bleibt. Ganz besonders vorteilhaft ist es, wenn eine wäßrige Lösung mit 5-15 % Weinsäure verwendet wird; die Aushärtezeit wird damit um mehr als 10 Minuten auf etwa 1-2 Minuten verkürzt.

Nach einer vorteilhaften Ausführungsform des erfindungsgemäßen Verfahrens wird das Ahrühren in der Weise durchgeführt, daß zunächst das Calciumaluminiumsilikatglas und/oder Calciumaluminiumfluorsilikatglas und das Polymer und/oder Copolymer einer ungesättigten Carbonsäure angerührt und dann der als Abbindestarter dienende Chelatbildner in Wasser bzw. der obengenannten wäßrigen Weinsäurelösung unter

fortgesetztem Anrühren zugesetzt werden. Dabei handelt es sich um ein anderes Vorgehen als das für die Zahnheilkunde übliche. In der Zahnheilkunde kommt es darauf an, den Glasionomer-Zement gamma-steril zu machen, was aber für die Mittelohr- und

Unfallchirurgie sowie die Rekonstruktion der hinteren Gehörgangswand nicht erforderlich ist. Die genannte vorteilhafte Ausführungsform des erfindungsgemäßen Verfahrens hat den Vorteil, daß die Erhärtung des Glasionomer-Zementes schneller (im allgemeinen 1 Minute) vonstatten geht und ferner mit einfacheren Vorrichtungen gearbeitet werden kann. Da für die genannten Anwendungen die Gamma-Sterilität nicht erforderlich ist, ist deren Fehlen unerheblich.

Die Erfindung und ihre Anwendung werden an Hand des folgenden Beispieles in Verbindung mit den beigefügten Zeichnungen näher erläutert.

Beispiel

In den Figuren zeigen:

Abb. 1     eine Glasionomer-Zement-Manschette bei narbiger Überbrückung eines Kleindefektes im kleinen Gelenk,

Abb. 2     eine Columella-Fixierung am Steigbügel und

Abb. 3     eine Fixierung einer Steigbügelprothese am Amboß mit Glasionomer-Zement.

Für chronische Mittelohrentzündungen sind Trommelfellperforationen und Höreinbußen infolge eines Kettendefektes im kleinen Gelenk charakteristisch. Während der Trommelfellverschluß mit den heutigen Techniken praktisch immer gelingt, mißrät die Kettenrekonstruktion im kleinen Gelenk sehr oft. Die Hörverbesserung ist nur vorübergehend, da später der nach außen gerichtete Narbenzug des eingelegten Trommelfelles erneut zu einer Unterbrechung im kleinen Gelenk führt. Der Patient hört gleich schlecht wie vor der Operation. Mit Glasionomer-Zement können stabile Verbindungen im Bereich des kleinen Gelenkes geschaffen werden. Bei Defekten kann mit Glasionomer-Zement eine stabile Manschette geschaffen werden, die den Schall sicher überträgt. Bei größeren Defekten, bei welchen die Entnahme des Amboß notwendig ist, kann der Amboßrest sicher mit Glasionomer-Zement am Steigbügel fixiert werden, ohne daß eine Abwanderung durch Narbenzug zu befürchten ist. Glasionomer-Zement eignet sich zur Fixierung von Steigbügelprothesen am Anboß.

Ein bisher nicht befriedigend gelöstes Problem in der tympanoplastischen Versorgung des kleinen Gelenkes bieten Kettenunterbrechungen oder Arrosionen des langen Amboßfortsatzes im Rahmen einer chronischen Otitis media.

Bisher übliche Operationsmethoden sind in diesem Fall sehr invasiv: Abbau der Gehörknöchelchenkette, Amboßinterposition, Columellabbildung oder Einsatz eines Interponats. Auch bei zunächst guten Hörgewinnen kommt es leider allzuoft durch Verschieben, Abgleiten oder Abheben des Interponates (Narbenzug) zu einer Verschlechterung des Ergebnisses. Eine Fixation mit Fibrinkleber ist wegen Resorption und der Elastizität des Materiales unbefriedigend.

Es soll ermöglicht werden, Defektevon 1-2 mm, aber auchbis zu 10 mm, bei Kettenunterbrechungen dauerhaft und befriedigend zu überbrücken. Weiterhin sollte damit eine feste Verbindung zwischen Steigbügel und Columella möglich sein.

Es wurde eine erfindungsgemäße Operationsnadel verwendet. Zu deren Herstellung wurde eine wäßrige Anmischflüssigkeit eines xenogenen Glasionomer-Materiales aus den folgenden Bestandteilen verwendet.

Mischung I (fest)

A) 83,0 Gew.-% Natriumcalciumaluminiumfluorsilikatglas aus:

175 Gew.-Teile $SiO_2$ ,

100 Gew.-Teile $Al_2O_3$ ,

207 Gew.-Teile $CaF_2$ ,

30 Gew.-Teile $Na_3AlF_6$ ,

32 Gew.-Teile $AlF_3$ ,

60 Gew.-Teile $AlPO_4$ ,

B) 17,0 Gew.-% Poly-(acryl/malein)-säure mit einem Verhältnis der Acrylsäure- und Maleinsäureeinheiten von 1 : 1 ,

Mischung II (Flüssigkeit)

C) 17,0 Gew.-% Weinsäure in Lösung

D) 83,0 Gew.-% Wasser .

Zunächst wurde die Mischung I mit der Operationsnadel angerührt und danach unter fortgesetztem Anrühren die Mischung II zugesetzt, bis an der Nadel ein kleiner Tropfen hängenblieb, der dem Operateur übergeben wurde. Bei dieser Anmischung bildete sich in einem ersten Reaktionsschritt ein Calciumpolycarboxylat, das noch relativ empfindlich auf Wasser reagierte. Erst die Bildung von Aluminiumkomplexen, die mehr Zeit in Anspruch nahm, führte zu einer kompletten Aushärtung. Je höher der Pulveranteil in einer vorgegebenen Flüssigkeitsmenge (Polycarbonsäurelösung) war, umso größer war die Härte und die Abrasionsfestigkeit und umso geringer die Löslichkeit.

Nach Darstellung der Kette und sorgfältigster Blutstillung wurde durch permanenten Luftzug ein besonders trockener Situs geschaffen. Erfahrungsgemäß ging etwa 1 Minute nach Anmischbeginn das Material in eine zähe, klebrige Konsistenz über. Die Masse haftete als Tropfen gut an der Operationsnadel, mit der eine Applikation am vorgesehenen Ort möglich war.

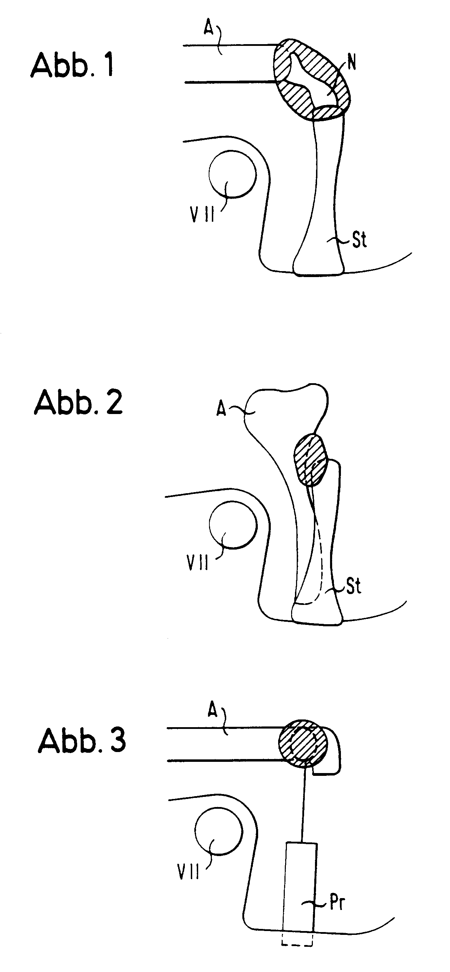

Bei Kettenunterbrechung bis 10 mm wurde der Tropfen am langen Amboßschenkel aufgebracht, in den Defekt gezogen und am Stapesköpfchen abgestrichen (Abb. 1). Bei Columella-Bildung nach Wullstein wurde der Tropfen zwischen Interponat und Stapesköpfchen beim Stapesschenkel abgestrichen (Abb. 2).

Die Stapedotomie wurde in der von Fisch beschriebenen Weise durchgeführt. Nach Schutz der Fußplattenperforation mit Faszie konnte anschließend die eingeklemmte Drahtschlinge am Amboß mit dem Glasionomer-Zement ummantelt werden (Abb.3).

Innerhalb 6 Monate wurden 12 Patienten mit Luxationen im kleinen Gelenk sowie 3 Stapedotomien in der beschriebenen Form versorgt. Präoperativ und 3 Wochen nach dem Eingriff wurde jeweils ein Audiogramm durchgeführt.

Die Ergebnisse der prä- und postoperativen audiometrischen Diagnostik ergaben eine durchschnittliche Verbesserung der Schalleitung von 25 dB. Am eindruckvollsten war die Verbesserung der "air-bone-gap"

EP 0 406 390 B1

nach Überbrückung eines narbigen Defektes am kleinen Gelenk. Hier betrug die postoperative Schalleitung 0 bis 15 dB.

Drei in der beschriebenen Form fixe Drahtschleifen am Amboß bei Stapedotomien ergaben postoperative "air-bone-gaps" von 0 bis 10 dB Innenohrabfall.

Die erfindungsgemäße Operationsnadel ergänzt in der Chirurgie des kleinen Gelenkes die bisherigen Mittel bei gehörverbessernden Operationen.

Eine neue bedeutende Art der Verwendung ist die Versorgung von Defekten des kleinen Gelenkes. Die schallübertragende Funktion der Gehörknöchelchenkette konnte so voll wieder hergestellt werden.

Im Laufe der klinischen Anwendung zeigte sich eine zusätzliche Möglichkeit der Fixierung eines zwischen Trommelfell und Fußplatte interponierten Incus am Stapesköpfchen. Die bisherigen klinischen Resultate lassen den Schluß zu, daß ein Abgleiten, Abheben oder Verschieben des Interponates bleibend verhindert werden kann.

Eine Prothesenlockerung bei Stapedotomien der Drahtschleife am langen Amboßfortsatz konnte offensichtlich durch Ummantelung mit dem Glasionomer-Zement der erfindungsgemäßen Operationsnadel verhindert werden. Bei allen derart versorgten Stapedotomien ergab sich ein optimaler bleibender postoperativer Hörgewinn, weil die Verbindung auch über lange Zeit stabil bleibt.

Der Umgang mit dem Material erforderte größtmögliche Sorgfalt. Ein Kontakt des Glasionomer-Zements mit dem ovalen Fenster, dem Zugang zum Innenohr, mußte wegen der anfänglichen Acidität unbedingt vermieden werden. Bisher traten keine postoperativen Innenohrschäden auf, auch nicht bei Stapedotomien.

Nach den bisher vorliegenden Ergebnissen kann auch im Mittelohr von einer guten Gewebeverträglichkeit ausgegangen werden, wie dies nach den Erfahrungen in der Zahnmedizin zu erwarten war.

**Patentansprüche**

1. Operationsnadel für die Mittelohr- und Unfallchirurgie sowie die Rekonstruktion der hinteren Gehörgangswand, dadurch gekennzeichnet, daß an ihr ein Tropfen einer wäßrigen Anmischung eines zu Glasionomer-Zement erhärtbaren Glasionomer-Materiales hängt.

2. Operationsnadel nach Anspruch 1, dadurch gekennzeichnet, daß die Tropfengröße 0,5 bis 2,0 mm beträgt.

3. Operationsnadel nach Anspruch 1 oder 2, dadurch gekennzeichnet, daß das Glasionomer-Material aus pulverförmigem Calciumaluminiumsilikatglas und/oder Calciumaluminiumfluorsilikatglas, gegebenenfalls mit einem Gehalt an Natrium und/oder Phosphor, aus einem Polymer und/oder Copolymer einer, ungesättigten Carbonsäure und aus einem Chelatbildner als Abbindestarter besteht.

4. Operationsnadel nach Anspruch 1 bis 3, dadurch gekennzeichnet, daß das Calciumaluminiumfluorsilikatglas aus

150 bis 200 Gew.-Teilen $SiO_2$ ,
75 bis 125 Gew.-Teilen $Al_2O_3$ ,
180 bis 240 Gew.-Teilen $CaF_2$ ,
20 bis 40 Gew.-Teilen $Na_3AlF_6$ ,
25 bis 40 Gew.-Teilen $AlF_3$ und
50 bis 70 Gew.-Teilen $AlPO_4$

besteht.

5. Operationsnadel nach Anspruch 1 bis 4, dadurch gekennzeichnet , daß das Polymer einer ungesättigten Carbonsäure, gegebenenfalls halogensubstituierte, Polyacrylsäure, gegebenenfalls halogensubstituierte, Polymethacrylsäure oder Polymaleinsäure und das Copolymer einer ungesättigten Carbonsäure ein solches von Acrylsäure und Methacrylsäure, Acrylsäure und Itaconsäure oder Acrylsäure und Maleinsäure oder Methacrylsäure und Itaconsäure oder Methacrylsäure und Maleinsäure oder Acrylsäure, Methacrylsäure und Itaconsäure oder Acrylsäure, Methacrylsäure und Maleinsäure oder Acrylsäure, Itaconsäure und Maleinsäure oder Methacrylsäure, Itaconsäure und Maleinsäure oder Acrylsäure, Methacrylsäure, Itaconsäure und Maleinsäure, die alle gegebenenfalls halogensubstituiert sein können, ist.

6. Operationsnadel nach Anspruch 1 bis 5, dadurch gekennzeichnet, daß der als Abbindestarter dienende Chelatbildner ein solcher mit 1 oder mehr Carboxyl- und/oder Hydroxygruppe(n) ist.

6

EP 0 406 390 B1

**7.** Operationsnadel nach Anspruch 1 bis 6, dadurch gekennzeichnet, daß der als Abbindestarter dienende Chelatbildner Weinsäure ist.

**8.** Operationsnadel nach Anspruch 1 bis 5, dadurch gekennzeichnet, daß der als Abbindestarter dienende Chelatbildner ein Metallchelat ist.

**9.** Operationsnadel nach Anspruch 1 bis 8, dadurch gekennzeichnet, daß die wäßrige Anmischflüssigkeit des Glasionomer-Materiales aus

35 bis 45 Gew.-% Calciumaluminiumsilikatglas und/oder Calciumaluminiumfluorsilikatglas, gegebenenfalls mit einem Gehalt an Natrium- und/oder Phosphor,

5 bis 15 Gew.-% Polymer und/oder Copolymer einer ungesättigten Carbonsäure,

5 bis 15 Gew.-% als Abbindestarter dienenden Chelatbildner und

35 bis 45 Gew.-% Wasser

besteht.

**10.** Operationsnadel nach Anspruch 9, dadurch gekennzeichnet, daß das Calciumaluminiumfluorsilikatglas ein Natriumcalciumaluminiumfluorsilikat-Glas, das Copolymer einer ungesättigten Carbonsäure Poly-(acryl/malein)-säure und der als Abbindestarter dienende Chelatbildner Weinsäure ist.

**11.** Verfahren zur Herstellung der Operationsnadel nach Anspruch 1 bis 10, dadurch gekennzeichnet, daß man das pulverförmige Calciumaluminiumsilikatglas und/oder Calciumaluminiumfluorsilikatglas, das Polymer und/oder Copolymer einer ungesättigten Carbonsäure und den als Abbindestarter dienenden Chelatbildner in Wasser mit einer Operationsnadel anrührt, bis an der letzteren ein Tropfen hängen bleibt.

**12.** Verfahren nach Anspruch 11, dadurch gekennzeichnet, daß man das Anrühren in der Weise durchführt, daß man zunächst das Calciumaluminiumsilikatglas und/oder Calciumaluminiumfluorsilikatglas und das Polymer und/oder Copolymer einer ungesättigten Carbonsäure anrührt und dann den als Abbindestarter dienenden Chelatbildner in Wasser unter fortgesetztem Anrühren zusetzt.

## Claims

**1.** Surgical needle for surgery of middle ear and surgery of accident caused injuries, and for reconstruction of the rear auditory canal characterized in that it carries a drop of an aqueous mixture of a glass ionomer material being hardenable to glass ionomer cement.

**2.** Surgical needle according to claim 1, characterized in that the size of the drop is ranging between 0.5 and 2.0 mm.

**3.** Surgical needle according to claim 1 or 2, characterized in that the glass ionomer material consists of a powder of calcium aluminium silicate glass and/ or calcium aluminum fluorosilicate glass, optionally containing sodium and/ or phosphorus, of a polymer and/ or copolymer of an unsaturated carboxylic acid, and of a chelating agent as setting starter.

**4.** Surgical needle according to claim 1 to 3, characterized in that calcium aluminum fluorosilicate glass consists of

150 to 200 parts by weight $Si\,O_2$ ,

75 to 125 parts by weight $Al_2O_3$ ,

180 to 240 parts by weight $Ca\,F_2$ ,

20 to 40 parts by weight $Na_3Al\,F_6$ ,

25 to 40 parts by weight $Al\,F_3$ et,

50 to 70 parts by weight $Al\,PO_4$ .

**5.** Surgical needle according to claim 1 to 4, characterized in that the polymer of the unsaturated carboxylic acid is, optionally halogene substituted, polyacrylic acid, optionally halogene substituted, polymethacrylic or polymaleic acid, and the copolymer of an unsaturated carboxylic acid is a copolymer of acrylic acid and methacrylic acid, acrylic acid and itaconic acid, or acrylic acid and maleic acid, or methacrylic acid and itaconic acid, or methacrylic acid and maleic acid, or acrylic acid,

7

methacrylic acid and itaconic acid, or acrylic acid, methacrylic acid and maleic acid, or acrylic acid, itaconic acid and maleic acid, or methacrylic acid, itaconic acid and maleic acid, or acrylic acid, methacrylic acid, itaconic acid and maleic acid, all those being optionally halogene substituted.

6. Surgical needle according to claim 1 to 5, characterized in that the chelating agent serving as setting starter has at least 1 carboxyle and/ or hydroxyle group.

7. Surgical needle according to claim 1 to 6, characterized in that the chelating agent serving as setting starter is tartric acid.

8. Surgical needle according to claim 1 to 5, characterized in that the chelating agent serving as setting starter is a metal chelate.

9. Surgical needle according to claim 1 to 8, characterized in that the aqueous mixture of the glass ionomer material consists of

35 to 45 wt. % calcium aluminium silicate glass and/ or calcium aluminum fluorosilicate glass, optionally containing sodium and/ or phosphorus
5 to 15 wt. % polymer and/ or copolymer of an unsaturated carboxylic acid
5 to 15 wt. % chelating agent serving as setting starter and
35 to 45 wt. % water

10. Surgical needle according to claim 9, characterized in that the calcium aluminium fluorosilicate glass is a sodium calcium aluminium fluorosilicate glass, the copolymer of an unsaturated carboxylic acid is poly-(acrylic/ maleic) acid, and the chelating agent serving as setting starter is tartric acid.

11. Process for obtaining the surgical needle according to claim 1 to 10, characterized in that the powder of calcium aluminium silicate glass and/ or calcium aluminum fluorosilicate glass, the polymer and/ or copolymer of an unsaturated carboxylic acid, and the chelating agent as setting starter in aqueous solution are stired together using a surgical needle, until a drop sticks to the needle.

12. Process according to claim 11, characterized in that the stiring is realized in a way that first the calcium aluminium silicate glass and/ or calcium aluminum fluorosilicate glass is mixed with the polymer and/ or copolymer of an unsaturated carboxylic acid, and later is added, while continuing to stir, the chelating agent as setting starter in aqueous solution.

**Revendications**

1. Aiguille chirurgicale pour la chirurgie de l' oreille moyenne et la chirurgie des blessures, ainsi que la reconstruction de la cloison auditif postérieure, caractérisée en ce qu' elle porte une goutte d' une mélange aqueuse d'une matière de ionomère de verre étant durcissable de façon à donner un ciment de ionomère de verre.

2. Aiguille chirurgicale selon la revendication 1, caractérisée en ce que la dimension des gouttes est comprise entre 0.5 mm et 2.0 mm.

3. Aiguille chirurgicale selon la revendication 1 ou 2, caractérisée en ce que la matière de ionomère de verte consiste d' un poudre de verre au calcium-aluminium-silicate et/ ou de verre au calcium-aluminium-fluor-silicate, en option contenant sodium et/ ou phosphor, d' un polymère et/ ou d' un copolymère d' un acide carboxylique non saturé, et d' un agent chélateur comme déclencheur du durcissement.

4. Aiguille chirurgicale selon la revendication 1 à 3, caractérisée en ce que le verre au calcium-aluminium-fluor-silicate consiste de
150 à 200 % en poids $Si O_2$,
75 à 125 % en poids $Al_2 O_3$,
180 à 240 % en poids $Ca F_2$,
20 à 40 % en poids $Na_3 Al F_6$,
25 à 40 % en poids $Al F_3$, et

50 à 70 % en poids Al PO$_4$.

5. Aiguille chirurgicale selon la revendication 1 à 4, caractérisée en ce que le polymère d' un acide carboxylique non saturé est un acide polyacrylique en option halogène-substitué, un acide polyméthacrylique ou polymaléique en option halogène-substitué, et le copolymère d'un acide carboxylique non saturé est un copolymère d' un acide acrylique et d' un acide méthacrylique, d' un acide acrylique et d' un acide itaconique, ou d' un acide acrylique et d' un acide maléique, ou d' un acide méthacrylique et d' un acide itaconique, ou d' un acide méthacrylique et d' un acide maléique, d' un acide acrylique, d' un acide méthacrylique et d' un acide itaconique, ou d' un acide acrylique, d' un acide méthacrylique et d' un acide maléique, ou d' un acide acrylique, d' un acide itaconique et d' un acide maléique, ou d' un acide méthacrylique, d' un acide itaconique et d' un acide maléique, ou d' un acide acrylique, d' un acide méthacrylique,d' un acide itaconique et d' un acide maléique, qui peuvent en option tout être halogène-substitués.

6. Aiguille chirurgicale selon la revendication 1 à 5, caractérisée en ce que l' agent chélateur servant comme déclencheur du durcissement comprend au moins 1 groupe carboxyl et/ ou hydroxyle.

7. Aiguille chirurgicale selon la revendication 1 à 6, caractérisée en ce que l' agent chélateur servant comme déclencheur du durcissement est acide tartrique.

8. Aiguille chirurgicale selon la revendication 1 à 5, caractérisée en ce que l' agent chélateur servant comme déclencheur du durcissement est un chélate métallique.

9. Aiguille chirurgicale selon la revendication 1 à 8, caractérisée en ce que le mélange aqueuse de la matière de ionomère de verte consiste de

35 à 45 % en poids de verre au calcium-aluminium-silicate et/ ou d' un verre au calcium-aluminium-fluor-silicate, en option contenant sodium et/ ou phosphor,

5 à 15 % en poids d' un polymère et/ ou d'un copolymère d' un acide carboxylique non saturé,

5 à 15 % en poids d' un agent chélateur servant comme declencheur du durcissement et

35 à 45 % en poids d' eau.

10. Aiguille chirurgicale selon la revendication 9, caractérisée en ce que le verre au calcium-aluminium-fluor-silicate est un verre au sodium-calcium-aluminium-fluor-silicate, le copolymère d' un acide carboxylique non saturé est un acide poly-(acrylique/maléique), et l' agent chélateur servant comme déclencheur du durcissement est un acide tartrique.

11. Procédé de production de l' aiguille chirurgicale selon la revendication 1 à 10, caractérisée en ce qu' on remue, au moyen d' une aiguille chirurgicale, le poudre de verre au calcium-aluminium-silicate et/ ou de verre au calcium-aluminium-fluor-silicate, le polymère et/ ou le copolymère d' un acide carboxylique non saturé, et l' agent chélateur étant delayé dans de l'eau et servant comme déclencheur du durcissement, jusqu' à ce qu' une goutte s' accroche à l' aiguille.

12. Procédé selon la revendication 11, caractérisée en ce que la préparation de la mélange est effectué de la manière qu' on remue d' abord le verre au calcium-aluminium-silicate et/ ou le verre au calcium-aluminium-fluor-silicate avec le polymère et/ ou le copolymère d' un acide carbonique non-saturé, et ensuite on ajoute, en continuant d' agiter, l' agent chélateur étant delayé dans de l'eau et servant comme déclencheur du durcissement.

Abb. 1

Abb. 2

Abb. 3